**Europäisches Patentamt**

**European Patent Office**

**Office européen des brevets**

(19)

(11) Publication number : **0 532 548 B1**

# EUROPEAN PATENT SPECIFICATION

(12)

(45) Date of publication of patent specification :
**10.08.94 Bulletin 94/32**

(51) Int. Cl.$^5$ : **C07C 6/12**

(21) Application number : **91909982.0**

(22) Date of filing : **16.05.91**

(86) International application number :
**PCT/US91/03409**

(87) International publication number :
**WO 91/18849 12.12.91 Gazette 91/28**

(54) **TRANSALKYLATION IN THE PRESENCE OF A CATALYST SLURRY.**

(30) Priority : **08.06.90 US 535048**

(43) Date of publication of application :
**24.03.93 Bulletin 93/12**

(45) Publication of the grant of the patent :
**10.08.94 Bulletin 94/32**

(84) Designated Contracting States :
**DE FR GB IT NL**

(56) References cited :
**WO-A-89/12613**
**GB-A- 839 501**
**US-A- 4 179 473**

(73) Proprietor : **ABB LUMMUS CREST INC.**
**1515 Broad Street**
**Bloomfield New Jersey 07003 (US)**

(72) Inventor : **SUCIU, George, Dan**
**417 Prospect Street**
**Ridgewood, NJ 07450 (US)**
Inventor : **PAUSTIAN, John, Earle**
**38 Adams Drive**
**Whippany, NJ 07981 (US)**
Inventor : **KWON, Joon, Taek**
**142 Derby Drive**
**Freehold, NJ 07728 (US)**

(74) Representative : **Rupprecht, Klaus, Dipl.-Ing.**
**c/o ABB Patent GmbH,**
**Postfach 10 03 51**
**D-68128 Mannheim (DE)**

EP 0 532 548 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the transalkylation of polyalkylbenzenes in the presence of a transalkylation catalyst. More particularly, this invention relates to the transalkylation of polyalkylbenzenes in the presence of a catalyst slurry.

Transalkylation of polyalkylbenzenes typically takes place in the liquid phase by contacting a mixture of polyalkylbenzenes and excess benzene with a catalyst. The catalyst may be, for example, an acidic zeolite or a supported acid catalyst such as a supported phosphoric acid catalyst. WO 89/12613 describes a process for the transalkylation of polyalkylbenzenes to monoalkylbenzenes in a reactor in the presence of a catalyst. The process comprises transalkylating a feed comprising polyalkylbenzenes, preferably benzene and polyalkylbenzenes, in a transalkylation reactor, in the presence of hydrogen gas. Such a process increases catalyst life and permits the use of a feed to the transalkylation reactor having a lower benzene to alkyl groups ratio. Examples of transalkylation reactions are the transalkylation of polyethylbenzenes, such as diethylbenzenes and triethylbenzenes, to ethylbenzene, and of diisopropylbenzene and triisorpropylbenzene to cumene. Such reactions typically take place at a B/A ratio (benzene to alkyl groups ratio) of from 5 to 20. The "B" term represents all of the benzene rings (moles) in the reaction mixture, whether substituted or not. The "A" term represents all of the alkyl chains (moles) present in the reaction mixture, irrespective of whether there are one or more alkyl groups per benzene ring. At B/A ratios below 7, the rate of deactivation of catalyst is excessive. At B/A ratios of 7 or higher, catalyst lives of one year or more may be obtained. The weight hourly space velocity (WHSV) of such reactions is of the order of 2 hr.$^{-1}$.

As the time on stream for a given batch of catalyst increases, its deactivation will lead to dropping conversions of the polyalkylbenzenes. The transalkylation reaction is usually started with a new batch of catalyst at a temperature which, at the space velocity practiced, will result in a conversion of the polyalkylbenzenes of approximately 70%. As operating conditions are maintained constant, conversion will drop gradually to a value of about 50%. When conversion of polyalkylbenzenes to monoalkylbenzenes is about 50%, the reaction temperature is increased in order to bring conversion back to the 70% level. The temperature may be increased further, periodically as required, maintaining the conversion within the range of 50% to 70%. The reaction is stopped and the catalyst is regenerated or replaced when a temperature is reached at which either no increase in conversion occurs, or side reactions leading to undesired products become predominant. The frequency of reactor shutdowns increases with the level of conversion achieved and space velocity. The frequency of reactor shutdowns decreases with the decrease in the B/A ratio.

In accordance with an aspect of the present invention, there is provided an improvement in a process for the transalkylation of a feed comprising at least one polyalkylbenzene in a reactor in the presence of a transalkylation catalyst to produce at least one monoalkylbenzene. The improvement comprises transalkylating the at least one polyalkylbenzene in the presence of the transalkylation catalyst wherein the transalkylation catalyst is provided as a slurry of catalyst particles.

The term "slurry," as used herein, means that the catalyst is provided as a suspension of solid particles in the reaction medium.

In accordance with one embodiment, the catalyst may be a zeolite catalyst. Zeolite catalysts which may be employed include, but are not limited to, Zeolite Y, ZSM-5, Zeolite-Beta, Omega zeolites, Zeolite X, mordenite, and chabazite.

In accordance with another embodiment, the catalyst may be a supported acid catalyst. Examples of supported acid catalysts which may be employed include aluminum chloride, phosphoric acid catalysts, and BF$_3$/HF.

The catalyst may be of a particle size having a diameter of from 0.1 micron to 10 mm, preferably from 0.1 micron to 1 mm, most preferably from 0.1 micron to 200 microns. Although the scope of the present invention is not to be limited to any theoretical reasoning, at such particle sizes, the time required for the diffusion of the reagents to the active sites of the catalyst, and of the products from the active sites, is much reduced in comparison to those needed when catalysts of conventional sizes are used. A significant increase of the reaction rate and of reactor productivity is achieved when catalysts having such particle sizes are employed.

In a preferred embodiment, the transalkylation reaction is performed in plug flow. This plug flow may be achieved either by employing a reactor with a high length diameter ratio or a series of Continuous Stirred Tank Reactors or CSTR's. The catalyst particles may be suspended in the reactor either due to mechanical mixing within the reactor or by forced circulation of the liquid which entrains catalyst particles of the slurry. To prevent entrainment of the catalyst particles in the slurry, the reactor may be equipped with filters, settling devices, hydrocyciones, or centrifuges. The reactor may also be in the shape of a loop provided with means to pump the slurry continuously around the loop and such a reactor may be equipped with means to introduce reagents and to remove the reaction mixture from the system. In order to promote plug flow, the reactor may be provided with packing devices which reduce the extent of transverse mixing as well as that of axial mixing.

By providing a slurry of catalyst particles, one may withdraw periodically small portions of catalysts, or one may remove continuously a small stream of catalyst from the reaction system without the need to discontinue the reaction operation. In addition, small portions of fresh or regenerated catalyst, or an adequate, continuous stream cf fresh or regenerated catalyst may be introduced into the reactor system in order to compensate for the amount withdrawn. Thus, desired conversion levels may be maintained constant over indefinite periods of time.

Transalkylation reactions which may be employed include the conversion of polyethylbenzenes, such as diethylbenzene and triethylbenzenes, to ethylbenzene, and the conversion of polyisopropylbenzenes, such as diisopropylbenzene and triisopropylbenzene, to cumene. It is to be understood, however, that the scope of the present invention is not limited to such conversions.

The transalkylation process of the present invention enables one to carry out transalkylation reactions at lower benzene-to-alkyl groups ratios and at higher space velocities, which results in increased reactor productivities.

The transalkylation process of the present invention may be carried out at a temperature of from 120°C to 360°C, preferably from 170°C to 270°C, and at a pressure of from 6,9 to 137,9 bar (100 to 2,000 psig), preferably from 27,6 to 41,4 bar (400 to 600 psig). The transalkylation process may also be carried out at a B/A (mol/mol) ratio of from 2 to 20, preferably from 3 to 10, and at a weight hourly space velocity (WHSV) of from 0.1 to 50, preferably from 3 to 30 $hr^{-1}$.

The invention will now be described with respect to the following examples; however, the scope of the present invention is not intended to be limited thereby.

For purposes of the following examples, the following definitions of terms are given herein.

B/E (mol/mol) - The ratio of the sum (moles) of all benzene rings, substituted or not, present in the feed to the reactor, to the sum (moles) of all ethyl side chains, irrespective of what rings to which they are bound.

B/P (mol/mol) - The ratio of the sum (moles) of all benzene rings, substituted or not, present in the feed to the reactor, to the sum (moles) of all isopropyl side chains, irrespective of what rings to which they are bound.

Conversion (ethyl) - Moles of ethyl groups which have left molecules of diethylbenzene (DEB) and triethylbenzene (TEB) present in the feed, divided by the moles of ethyl groups in the DEB and TEB present in the product.

Selectivity (ethyl) - Moles of ethyl groups bound in the ethylbenzene (EB) present in the product, divided by the moles of ethyl groups bound in the DEB and TEB disappeared from the feed.

In the following examples, a vertical metal tube reactor is placed in a fluidized sand bath for temperature control. The reactor is filled with an acidic zeolite (Zeolite Y) shaped as extrudates. A metering pump feeds the reagents, polyalkylbenzenes in excess benzene, through the bottom of the reactor. The product mixture leaves the reactor, passes through a pressure control device, and is collected and analyzed. The reactor pressure is set so as to maintain the reactor content in the liquid phase.

Example 1 (Comparative)

A liquid rich in DEB and TEB was mixed with benzene to give a solution having a B/E ratio of 11 and was fed to the reactor at a weight hourly space velocity of 2.3 g/g catalyst x hour. At the beginning of the reaction, the temperature was increased periodically in order to maintain the conversion of DEB in the range of 60% to 70%. At the end of 5,616 hours on stream the total ethylbenzene production was 855 kg EB/kg catalyst, or the average catalyst production rate was 0.15 kg EB/kg catalyst x hr.

In a similar reaction, at a B/E ratio of 6, the activity of the catalyst was lost after about 400 kg EB/kg catalyst were produced. The composition of the feed and effluent streams is given in Table 1 below.

TABLE 1
Transalkylation in Fixed Bed Reactor
Concentration (%)

| Component | Feed | Product | Conversion |
|---|---|---|---|
| $C_4$-$C_6$ Aliphatics | 0.05 | 0.05 | |
| Benzene | 92.84 | 89.58 | |
| Toluene | - | 0.008 | |
| Ethylbenzene | 0.04 | 7.76 | |
| Propylbenzene | 0.03 | 0.007 | |
| Butylbenzenes | 0.09 | 0.08 | |
| Diethylbenzenes | 6.37 | 2.15 | 65.7 |
| $C_{11}$ Aromatics | 0.03 | 0.02 | |
| Triethylbenzenes | 0.45 | 0.21 | 52.0 |
| Diphenylethane | - | 0.09 | |
| High Boilers | 0.02 | 0.04 | |

At higher WHSV values (i.e., above 2.3), the conversion of diethylbenzene was below the acceptable value of 60%.

Example 2 - Transalkylation over Slurried Catalyst

The experimental setup is the same as that of Example 1, except that the reactor is fitted at both ends with filtering plugs made of sintered metal which prevent the passage of catalyst particles larger than 0.5 microns. The reactor contains a commercial Zeolite Y in acid form, with particle sizes smaller than 200 mesh. The volume of the dry settled catalyst occupies ½ of the reactor volume between the two sintered metal filters. In reaction conditions the catalyst bed will expand to occupy all the reactor volume available.

A transalkylation reaction was carried out using a stream rich in diethylbenzene and triethylbenzene obtained by fractionation, from the effluent of alkylation reactors. The stream was mixed with fresh benzene to obtain a B/E ratio of 3. The reaction was conducted at a WHSV of 20 hr. $^{-1}$. The temperature at the start of the reaction was 230°C (446°F). The DEB conversion of the feed and product streams are given in Table 2 below.

TABLE 2

DEB Transalkylation in Slurry Reactor

Concentration (wt. %)

| Component | Feed | Product | Conversion |
|---|---|---|---|
| $C_4$-$C_6$ | 0.05 | 0.05 | |
| Benzene | 74.83 | 65.82 | |
| Toluene | - | 0.01 | |
| Ethylbenzene | 0.05 | 24.36 | |
| Propylbenzene | 0.003 | 0.04 | |
| Butylbenzene | 0.38 | 0.32 | |
| Diethylbenzene | 24.25 | 8.37 | 65.5 |
| $C_{11}$ Aromatics | 0.21 | 0.17 | |
| Triethylbenzene | 0.11 | 0.54 | |
| Diphenylethane | 0.001 | 0.19 | |
| High Boilers | 0.07 | 0.12 | |

Because the initial concentration of TEB is below the equilibrium value for the reaction temperature, this compound is not consumed, but more is produced.

The average ethylbenzene production rate was 4.33 kg, EB/kg x hr. The catalyst lost its activity after 4,780 kg EB/kg catalyst were produced.

Example 3 - Transalkylation of DIPB (comparative)

The reactor and catalyst of Example 1 were used. The feed consisted of a mixture of benzene and isomers of DIPB so that the B/P (benzene to isopropyl groups) ratio was 7. The reactor was operated at a WHSV of 4 hr.$^{-1}$. During the reaction, the temperature was increased to between 230°C and 280°C as needed, in order to obtain a conversion of DIPB's in the range of 65% to 80%. The production rate for cumene varied in the range of 0.48-0.60 kg/kg hour. The catalyst was deactivated after 900 kg cumene/kg catalyst were produced.

Example 4 - Transalkylation of DIPB in the Presence of Slurried Catalyst

The reactor and catalyst of Example 2 were employed. A mixture of pure diisopropylbenzenes and benzene were fed to the reactor at a B/P ratio of 3 and at a WHSV of 20 hr.$^{-1}$. The temperature was increased to between 240°C and 300°C in order to maintain the conversion in the 60% to 73% range. The average production rate of cumene was 5.5 kg/kg x hour catalyst. The catalyst lost its activity when 3,360 kg cumene/kg catalyst were produced.

Advantages of the present invention include the ability to carry out the transalkylation of polyalkylbenzenes at lower benzene to alkyl groups ratios and at increased space velocities, thus resulting in increased reactor productivities. The present invention also provides for increased catalyst life, and the ability to replace spent catalyst continuously or intermittently without interrupting operation of the reactor.

It is to be understood, however, that the scope of the present invention is not to be limited to the specific embodiments described above The invention may be practiced other than as particularly described and still be within the scope of the accompanying claims.

## Claims

1.  In a process for the transalkylation of a feed comprising at least one polyalkylbenzene in a reactor in the presence of a transalkylation catalyst to produce at least one monoalkylbenzene, the improvement comprising:
    transalkylating said polyalkylbenzene (5) in the presence of said transalkylation catalyst wherein said transalkylation catalyst is provided as a slurry of catalyst particles.

2.  The process of Claim 1 wherein said catalyst is a zeolite catalyst.

3.  The process of Claim 1 wherein said catalyst is a supported acid catalyst.

4.  The process of Claim 1 wherein said catalyst has a particle size having a diameter of from 0.1 micron to 10 mm.

5.  The process of Claim 4 wherein said catalyst has a particle size having a diameter of from 0.1 micron to 1 mm.

6.  The process of Claim 5 wherein said catalyst has a particle having a diameter size of from 0.1 micron to 200 microns.

7.  The process of Claim 1 wherein said polyalkylbenzene (5) includes at least one of the group consisting of diethylbenzene and triethylbenzene.

8.  The process of Claim 7 wherein said monoalkylbenzene is ethylbenzene.

9.  The process of Claim 1 wherein said polyalkylbenzene (5) includes at least one of the group consisting of diisopropylbenzene and triisopropylbenzene.

10. The process of Claim 9 wherein said monoalkylbenzene is cumene.

11. The process of Claim 1 wherein said transalkylation is carried out at a temperature of from 120°C to 360°C.

12. The process of Claim 11 wherein said transalkylation is carried out at a temperature of from 170°C to 270°C.

13. The process of Claim 1 wherein said transalkylation is carried out at a pressure of from 6,9 to 137,9 (100 psig to 2,000 psig).

14. The process of Claim 13 wherein said transalkylation is carried out at a pressure of from 27,6 to 41,4 bar (400 psig to 600 psig).

15. The process of Claim 1 wherein said transalkylation is carried out at a benzene to alkyl groups mole ratio of from 2 to 20.

16. The process of Claim 15 wherein said transalkylation is carried out at a benzene to alkyl groups mole ratio of from 3 to 10.

17. The process of Claim 1 wherein said transalkylation is carried out at a weight hourly space velocity of from 0.1 hr.$^{-1}$ to 50 hr.$^{-1}$.

18. The process of Claim 17 wherein said transalkylation is carried out at a weight hourly space velocity of from 3 hr.$^{-1}$ to 10 hr.$^{-1}$.


**Patentansprüche**

1. Verbessertes Verfahren zur Transalkylierung eines Einsatzstoffes aus mindestens einem Polyalkylbenzol in einem Reaktor in Gegenwart eines Transalkylierungskatalysators unter Bildung mindestens eines Monoalkylbenzols, wobei die Verbesserung darin besteht, daß der Transalkylierungskatalysator als eine Aufschlämmung von Katalysatorteilchen vorliegt.

2. Verfahren nach Anspruch 1, worin der Katalysator ein Zeolith-Katalysator ist.

3. Verfahren nach Anspruch 1, worin der Katalysator ein saurer Trägerkatalysator ist.

4. Verfahren nach Anspruch 1, worin der Katalysator eine Teilchengröße mit einem Durchmesser von 0,1 Mikron bis 10 mm besitzt.

5. Verfahren nach Anspruch 4, worin der Katalysator eine Teilchengröße mit einem Durchmesser von 0,1 Mikron bis 1 mm besitzt.

6. Verfahren nach Anspruch 5, worin der Katalysator eine Teilchengröße mit einem Durchmesser von 0,1 Mikron bis 200 Mikron besitzt.

7. Verfahren nach Anspruch 1, worin das Polyalkylbenzol(gemisch) mindestens ein solches aus der Gruppe Diethylbenzol und Triethylbenzol amfaßt.

8. Verfahren nach Anspruch 7, worin das Monoalkylbenzol Ethylbenzol ist.

9. Verfahren nach Anspruch 1, worin das Polyalkylbenzol-gemisch mindestens einem solches aus der Gruppe Diisopropylbenzol und Triisopropylbenzol umfaßt.

10. Verfahren nach Anspruch 9, worin das Monoalkylbenzol Cumol ist.

11. Verfahren nach Anspruch 1, worin die Transalkylierung bei einer Temperatur von 120°C bis 360°C durchgeführt wird.

12. Verfahren nach Anspruch 11, worin die Transalkylierung bei einer Temperatur von 170°C bis 270°C durchgeführt wird.

13. Verfahren nach Anspruch 1, worin die Transalkylierung bei einem Druck von 6,9 bis 137,9 bar (100 psig bis 2 000 psig) durchgeführt wird.

**14.** Verfahren nach Anspruch 13, worin die Transalkylierung bei einem Druck von 27,6 bis 41,4 bar (400 psig bis 600 psig) durchgeführt wird.

**15.** Verfahren nach Anspruch 1, worin die Transalkylierung bei einem Molverhältnis von Benzol zu Alkylgruppen von 2 bis 20 durchgeführt wird.

**16.** Verfahren nach Anspruch 15, worin die Transalkylierung bei einem Molverhältnis von Benzol zu Alkylgruppen von 3 bis 10 durchgeführt wird.

**17.** Verfahren nach Anspruch 1, worin die Transalkylierung bei einer Katalysatorbelastung (WHSV) von 0,1 $h^{-1}$ bis 50 $h^{-1}$ durchgeführt wird.

**18.** Verfahren nach Anspruch 17, worin die Transalkylierung bei einer Katalysatorbelastung (WHSV) von 3 $h^{-1}$ bis 10 $h^{-1}$ durchgeführt wird.

**Revendications**

**1.** Dans un procédé de transalkylation d'une charge comprenant au moins un polyalkylbenzène dans un réacteur, en présence d'un catalyseur de transalkylation, pour produire au moins un monoalkylbenzène, l'amélioration comprenant :
la transalkylation desdits polyalkylbenzènes, en présence dudit catalyseur de transalkylation, ledit catalyseur de transalkylation étant fourni sous forme d'une suspension de particules de catalyseur.

**2.** Procédé selon la revendication 1, dans lequel ledit catalyseur est un catalyseur de type zéolithe.

**3.** Procédé selon la revendication 1, dans lequel ledit catalyseur est un catalyseur acide supporté.

**4.** Procédé selon la revendication 1, dans lequel ledit catalyseur a une granulométrie telle que le diamètre des particules soit de 0,1 micron à 10 mm.

**5.** Procédé selon la revendication 4, dans lequel ledit catalyseur a une granulométrie telle que le diamètre des particules soit de 0,1 micron à 1 mm.

**6.** Procédé selon la revendication 5, dans lequel ledit catalyseur a une granulométrie telle que le diamètre des particules soit de 0,1 micron à 200 microns.

**7.** Procédé selon la revendication 1, dans lequel lesdits polyalkylbenzènes englobent au moins un composé du groupe constitué par le diéthylbenzène et le triéthylbenzène.

**8.** Procédé selon la revendication 7, dans lequel ledit monoalkylbenzène est l'éthylbenzène.

**9.** Procédé selon la revendication 1, dans lequel lesdits polyalkylbenzènes englobent au moins un composé du groupe constitué par le diisopropylbenzène et le triisopropylbenzène.

**10.** Procédé selon la revendication 9, dans lequel ledit monoalkylbenzène est le cumène.

**11.** Procédé selon la revendication 1, dans lequel ladite transalkylation est réalisée à une température de 120°C à 360°C.

**12.** Procédé selon la revendication 11, dans lequel ladite transalkylation est réalisée à une température de 170°C à 270°C.

**13.** Procédé selon la revendication 1, dans lequel ladite transalkylation est réalisée sous une pression de 6,9 à 137,9 bars (100 psig à 2 000 psig).

**14.** Procédé selon la revendication 13, dans lequel ladite transalkylation est réalisée sous une pression de 27,6 à 41,4 bars (400 psig à 600 psig).

**15.** Procédé selon la revendication 1, dans lequel ladite transalkylation est réalisée avec un rapport molaire du benzène aux groupes alkyle de 2 à 20.

16. Procédé selon la revendication 15, dans lequel ladite transalkylation est réalisée avec un rapport molaire du benzène aux groupes alkyle de 3 à 10.

17. Procédé selon la revendication 1, dans lequel ladite transalkylation est réalisée à une vitesse spatiale horaire en poids de 0,1 h$^{-1}$ à 50 h$^{-1}$.

18. Procédé selon la revendication 17, dans lequel ladite transalkylation est réalisée à une vitesse spatiale horaire en poids de 3 h$^{-1}$ à 10 h$^{-1}$.